# EUROPÄISCHE PATENTSCHRIFT

(11) **EP 1 293 180 B1**
(45) Veröffentlichungstag und Bekanntmachung des Hinweises auf die Patenterteilung: **01.03.2006**
(21) Anmeldenummer: 01120459.1
(22) Anmeldetag: 28.08.2001
(51) Int. Cl.: A61F 2/44

(54) **Wirbelsäulenimplantat mit einstellbarer Höhe**
Spinal implant with adjustable height
Implant spinal avec hauteur régleable

(43) Veröffentlichungstag der Anmeldung: 19.03.2003
(73) Patentinhaber: Schröder, Johannes, 46325 Borken (DE)
(72) Erfinder: Schröder, Johannes, 46325 Borken (DE)

(56) Entgegenhaltungen:
- WO-A-02/09626
- US-A- 5 865 848
- US-A- 6 159 211

## Beschreibung

Die Erfindung bezieht sich auf Implantate, welche in der Wirbelsäulenchirurgie nach der Entfernung von Zwischenwirbelscheiben (Bandscheiben) oder Wirbelkörpern bzw. Teilen von Wirbelkörpern zur Wiederherstellung der Stabilität der Wirbelsäule eingesetzt werden

Implantate kommen in der Wirbelsäulenchirurgie in vielfältiger Weise zum Einsatz. Da der größere Anteil der axialen Belastung der Wirbelsäule durch die vorderen Elemente also die Wirbelkörper getragen wird, kommen Implantaten zum Ersatz dieser tragenden Funktion eine besondere Bedeutung zu. Bei der operativen Behandlung verschiedener Wirbelsäulenerkrankungen kommen solche Implantate zum Einsatz so z.B. nach der Entfernung von Halsbandscheiben wegen eines Bandscheibenvorfalles, nach der Entfernung von Wirbelkörpern wegen einer angeborenen oder erworbenen Enge des dahinter liegenden Wirbelkanals oder wegen eines tumorösen Befalls eines Wirbelkörpers. Mittels Implantat kann dann Statik und Funktion der Wirbelsäule wiederhergestellt werden. Da genaue Größe nicht vor sondern erst beim eigentlichen operativen Eingriff vom behandelnden Chirurgen eingeschätzt werden kann, ist im Normalfall eine größere Anzahl verschieden großer Implantate steril im Vorrat zu lagern.

Gegenstand der vorliegenden Erfindung ist ein in seiner Einbauhöhe variables Implantat. Implantate mit variabler Höhe bzw. der Höhengewinn durch das Stapeln von Implantaten sind bekannt Sie erfordern jedoch entweder komplizierte Mechanismen zur Höheneinstellung oder zusätzliche Elemente (Schrauben oder Platten) zur mechanischen Stabilisierung der Gesamtkonstruktion Eine der wesentlichen Forderungen bei der Entwicklung medizinischer Implantate ist ihre Robustheit gegenüber Störfaktoren und die (lebens-)langen Standzeiten bei vorher nicht abzuschätzender repetitiver Belastung.

Im Rahmen Recherche nach dem Stand der Technik sind andere unserer Entwicklung nahe kommende Bauformen erwähnenswert. Gregg S.Baker aus Geneva Florida ließ sich am 2.9.99 unter der US-Patentnummer US 5,865,848A einen dynamischen intervertebralen Spacer patentieren. Zwei identische Implantate werden longitudinal gegeneinander verschoben und ermöglichen dabei einen Höhengewinn. Der entscheidende Nachteil ist, daß sich damit auch die Länge des Implantates verändert.
Durch eine zirkuläre Anordnung der Abstandsflächen läßt wie in der älteren Patentschrift von Howard Cohen aus Metuchen New Jersey (International Publication Number WO 02/09626A1) vorgestellt, dieser Effekt ausschalten. Beide Implantate kommen jedoch im Gegensatz zu unserer Entwicklung nicht ohne zusätzliche Sicherungselemente gegen unbeabsichtigtes Verdrehen oder Verschieben aus.

Gegenstand der Erfindung ist in seiner Bauhöhe variables Implantat, welches ohne zusätzliche Elemente auskommt. Es besteht aus zwei identischen Teilen und erlaubt beim Zusammenbau der Komponenten abhängig von der axialen Winkelstellung zueinander einen Höhengewinn von 54% der Implantathöhe in mehreren Stufen.

Das erfindungsgemäße Implantat weist mehrere Vorteile auf Es liegt ein stabiles Implantat vor, welches ohne vorherige Paß- und Probeimplatate eingesetzt werden kann Bei zu geringer Vorspannung kann das gleiche Implantat in anderer Stellung montiert erneut eingesetzt werden. Zusätzliche Elemente zur Befestigung sind nicht notwendig. Das Risiko von Bruch, Lockerung oder Verlust dieser Elemente mit der Konsequenz einer Nachoperation des Patienten werden vermieden.
Die Konstruktion ist einfach. Im Zusammenhang mit neuen Krankheitsübertragungswegen durch Prionenerkrankungen (BSE, neue Variante der Kreutzfeld-Jakob-Erkrankung) werden höhere Anforderungen an Reinigung und Sterilisierbarkeit gestellt, so muß die weitestgehende Demontierbarkeit zur Reinigung möglich sein Komplizierte Implantate mit beweglichen Teilen sind nach gegenwärtig üblicher Sterilisationsbehandlung noch proteinkontaminiert und stellen ein potentielles Übertragungsrisiko dar. Nichtzuletzt ist das erfindungsgemäße Implantat universell. Der überwiegende Teil der vorderen Halsbandscheibenchirurgie notwendigen Implantatbauhöhen wird sich mit einem einzelnen Typ Implantat abdecken lassen.

Im folgenden wird die Erfindung anhand eines Beispiels und mit Bezug auf die beiliegenden Zeichnungen näher erläutert, wobei
Abbildung 1 die Schrägansicht eines erfindungsgemäßen Implantates vor dem Zusammenbau mit seinem um 180° gedrehten identischen Gegenpart und
Abbildung 2 die Montage des erfindunggemäßen Implantates mit seinem identischen Implantatpartner zum Gesamtimplantat in verschiedenen Implantathöheneinstellungen zeigt.

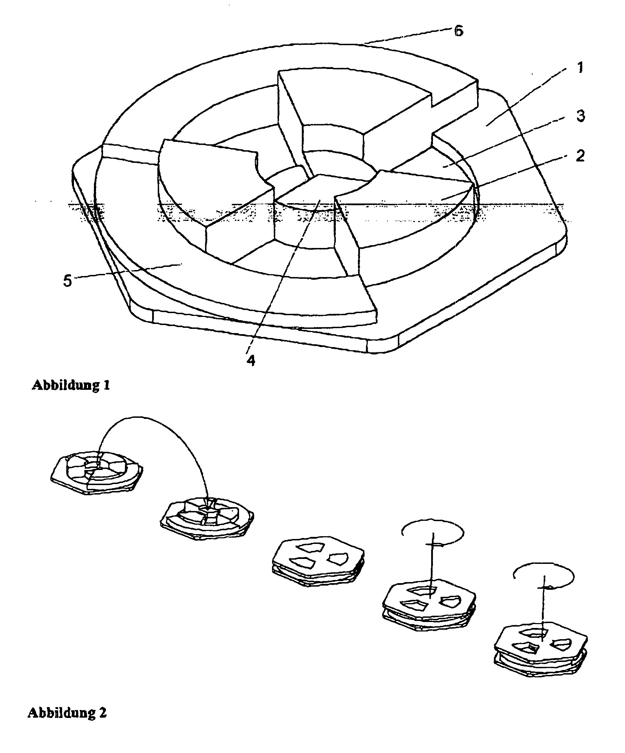

In Abbildung 1 ist das Wirbelsäulen- Implantat dargestellt. Es besteht aus dem Implantatkörper (1) welcher den Kontakt mit dem Knochen des angrenzenden Wirbelkörpers aufnimmt. Verbunden damit sind Führungselemente (2), welche in die entsprechenden Fuhrungsnuten (3) des identischen Implantatpartners (1) eingreifen und eine teleskopierende Bewegung ermöglichen. Die Führungsnuten enden in passenden Öffnungen des Implantatkörpers (3). In voll eingefahrerem Zustand schließen die Führungselemente mit der Grundplatte des Implantatkörpers ab Ein innerer(4) und ein äußerer Ring(5) mit treppenförmig angeordneten Abstandhaltern definiert die Höhe des Gesamtimplantates. Das Gesamtimplantat besteht aus zwei identischen Implantaten, ein weiteres Implantat (1) wird entgegengesetzt aufgesetzt. Durch das graduelle Verdrehen der beiden Implantatpartner vor dem Zusammenbau können verschiedene Bauhöhen erreicht werden. Formelemente auf Ober- bzw. Unterseite sichern das Implantat gegen Verschiebung im implantierten Zustand. Dargestellt ist ein Implantat mit drei Bauhöhen. Die Anzahl der Verstellmöglichkeiten definiert die Anzahl der Führungselemente und die Anzahl der Abstandhalter auf dem inneren (4) und äußeren (5) Höhendefinitionsring. Auch die äußere Implantatform ist davon abhängig, im vorliegenden Beispiel ergibt sich bei drei Verstellmöglichkeiten mit drei Führungselementen (2) und jeweils drei Abstandshaltern bei 0°, 120° und 240° auf den Höhendefinitionsringen (4,5) eine sechseckige Außenform. Analog dazu würde die 2-fache Höhenverstellung in jeweils 180° Drehung zwei Führungselemente, zwei Führungsnuten, zwei Abstandhalter und eine quadratische Grundfläche bedingen, die 4-fache Verstellung 4 Elemente jeden Typs und einen Oktaeder als Grundform. Die theoretisch mögliche einfache runde Grundform wird vermieden, da sie die Wirbelkörperhinterkante überragt. Eine Bohrung (6) in der äußeren Zirkumferenz dient zur Aufnahme eines Implantatsetzinstrumentes beim Einbringen des Implantates.

## Patentansprüche

1. Implantat mit einstellbarer Höhe bestehend aus zwei identischen Körpern, wobei jeder Körper mit mindestens zwei Führungselementen (2) und mit Führungsnuten (3) versehen ist und die Führungselemente des einen Körpers in die Führungsnuten des anderen Körpers (1) eingreifen und eine teleskopierende Bewegung ermöglichen, wobei ein innerer Ring (4) und ein außerer Ring (5) mit jeweils treppenförmig angeordneten Abstandhaltern die Höhe des Gesamtimplantates definieren und wobei durch graduelles Verdrehen der beiden Körper (1) vor dem Zusammenbau verschiedene Bauhöhen erreicht werden können.

2. Implantat mit einstellbarer Höhe gemäß Anspruch 1, **dadurch gekennzeichnet daß** zwei identische Körper durch Formschluß bedingt durch ihre axiale Rotationsstellung zueinander unterschiedliche Bauhöhen der Gesamtkonstruktion ergeben.

## Claims

1. Implant with adjustable height, comprising two identical bodies, each body being provided with at least two guide elements (2) and with guide grooves (3), and the guide elements of one body engaging in the guide grooves of the other body (1) and permitting a telescoping movement, an inner ring (4) and an outer ring (5) each having spacers arranged in a step formation and defining the total implant height, and different overall heights being able to be obtained by gradual twisting of the two bodies (1) before installation.

2. Implant with adjustable height according to Claim 1, **characterized in that** two identical bodies, positively engaged with one another, permit different heights of the overall construction as a result of their axial rotation position relative to one another.

## Revendications

1. Implant à hauteur réglable se composant de deux corps identiques, chaque corps étant pourvu d'au moins deux éléments de guidage (2) et de rainures de guidage (3) et les éléments de guidage d'un corps venant en prise dans les rainures de guidage de l'autre corps (1) et permettant un mouvement télescopique, une bague interne (4) et une bague externe (5) avec des éléments d'écartement disposés en forme d'escalier définissant la hauteur de l'implant total, et différentes hauteurs de construction pouvant être obtenues par rotation graduelle des deux corps (1) avant l'assemblage.

2. Implant à hauteur réglable selon la revendication 1, **caractérisé en ce que** deux corps identiques, du fait de leur engagement positif, produisent, par leur position en rotation axiale, différentes hauteurs de construction de la construction totale.
